# EUROPEAN PATENT SPECIFICATION

(11) **EP 3 407 961 B1**
(45) Date of publication and mention of the grant of the patent: **14.07.2021**
(21) Application number: 17743839.7
(22) Date of filing: 01.01.2017
(51) Int. Cl.: A61B 18/20, A61B 18/00, A61B 18/22, A61N 5/06, A61N 5/067

(54) **SKIN TREATMENT APPARATUS**
HAUTBEHANDLUNGSVORRICHTUNG
APPAREIL DE TRAITEMENT DE LA PEAU

(30) Priority: 25.01.2016 US 201662286458 P
(43) Date of publication of application: 05.12.2018
(73) Proprietor: Syneron Medical Ltd., 2069200 Yoqneam Illit (IL)
(72) Inventor: EISENMANN, Shmulik, 3713405 Pardes Chana-Karkur (IL); KADOSH, Itai, 9372102 Jerusalem (IL); KIDRON, Einat, 4061465 Tel Mond (IL); GOLAND, Vladimir, 7775236 Ashdod (IL); GABAY-MADER, Carmit, 2064301 Yoqneam Illit (IL)
(74) Representative: Nederlandsch Octrooibureau
(86) International application number: PCT/IL2017/050005
(87) International publication number: WO 2017/130185

(56) References cited:
- US-A- 6 149 644
- US-A1- 2008 287 930
- US-A1- 2010 063 490
- US-A1- 2010 063 490
- US-A1- 2013 261 606
- US-A1- 2013 261 606
- US-A1- 2013 345 541
- US-A1- 2013 345 541
- US-A1- 2015 025 513
- US-A1- 2015 025 513
- US-A1- 2015 230 863
- US-E- R E37 504
- US-E- R E37 504

## Description

### TECHNOLOGY FIELD

The present description relates to a system for aesthetic treatment of a human cutaneous and subcutaneous tissue and in particular for treatment of large segments of tissue.

### BACKGROUND

Tissue is frequently treated non-invasively by different energies delivered to the skin. Types of energies that may be found in use for skin treatment include ultra sound (US) energy, Radio Frequency (RF) energy, microwave (MW) radiation or radiation energy emitted by a source of light or heat. The skin treatment energy is coupled to the skin by an applicator. The spot-size of the applicator is the area of the interface for delivering the energy and it defines to some extent the segment of skin or tissue to which the skin treatment energy is transferred. In order to treat another skin segments, the applicator is repositioned or re-aligned across a larger segment of the skin and activated to couple treatment energy to this segment of skin. The size of a treated segment of skin varies from about 3x3mm² to about 30x30mm².

After applying treatment to a specific skin segment the remaining segments of the skin are treated by moving or repositioning the applicator across a larger skin segment. A caregiver providing the skin treatment manually repositions the applicator. Although the time of skin treatment energy delivery could be controlled, other parameters would much depend on the expertise of the caregiver such as treated area overlap, quality of the contact, pressure applied to the applicator etc... As a result, not all skin segments are treated uniformly and evenly.

The human or animal skin has a three-dimensional contour and in addition to the caregiver errors, the skin contour complicates proper applicator positioning on the skin and optimal coupling or delivery of tissue or skin affecting energy.

The skin treatment usually continues for tens of minutes (20 - 60 minutes) depending on the treatment area size and naturally causes some fatigue to the caregiver. Reliance on the caregiver expertise for repositioning of the applicator frequently causes some of the skin treated areas to receive a lower than desired portion of energy and be at a temperature lower than the optimal treatment temperature, while other skin areas could receive a larger than desired portion of energy and be at a temperature higher than the optimal treatment temperature. Relevant prior art is disclosed by US2010/063490A1, US2015/025513A1, US2013/261606A1, US RE37504E and US2013/345541A1.

### GLOSSARY

The term "skin" as used in the present disclosure includes the outer skin layers such as stratum corneum, dermis, epidermis, connective tissue and the deeper subcutaneous layers such as adipose tissue. The terms "tissue" or "skin" as used in the present disclosure have the same meaning and are used interchangeable through the text of the disclosure.

The term "skin treatment energy" as used in the present disclosure means electromagnetic energy delivered to the skin by a treatment energy application device.

The term "treatment energy source" may be a laser source, for example a semiconductor laser such as laser diode, VCSEL, an assembly of laser diodes or bars or a solid state laser such as an Nd:YAG or Alexandrite for example, or a fiber laser, or other laser source or sources. The treatment energy source may be a broad spectrum light source of either coherent or non-coherent radiation source such as a Xe, Kr, W, Quartz-Iodine lamps or a high power LED. In some examples the treatment energy source maybe microwave energy source or sources.

The term "scanning angle" as used in the present disclosure means half the angle between the extreme scanning beam locations on the skin of the incident treatment energy beam and the energy source or scanning mirror/deflector. In the case the energy source is a ultrasound phased array the "scanning angle" will mean half the angle between the extreme scanning locations of the incident treatment energy beam on the skin and the phase center of the array, which is the location that the radiation appears to emanate. The scanning angle defines the length of a treatment energy beam sweep on the skin. In some examples the scanning angle of the same scanning system could be a variable angle characterizing a shorter or a longer treatment energy beam sweep.

The term "incidence angle" as used in the present disclosure means an angle between the treatment energy beam and the skin at each of the locations the beam impinges the skin.

The term "tissue/skin affecting energy" or "treatment energy" as used in the present disclosure means energy capable of causing a change in the tissue including heating and affecting hair follicles, hair papillae, sebaceous glands, fat cells, blood vessels, connective tissue, or supporting such change. Such energy for example, may be optical radiation in visible or invisible part of electromagnetic spectrum. The exact parameters of the energy source such as power, fluence, wavelength, etc., may be chosen depending on the specific application and clinical effect to target tissue.

The term "target tissue temperature" as used in the present disclosure means temperature of the targeted tissue such as dermis, hair follicle, hair papillae, sebaceous glands, fat cells, blood vessel, pigmented lesion, adipose or deeper subcutaneous tissue. The temperature of the target tissue could be derived based on the temperature of the skin overlaying the target tissue.

The term "computer" as used in the present disclosure means a computer including a processing unit capable of receiving data or information, processing it, and delivering the data processing results to another device. As such, a computer may include, as nonlimiting examples, a personal computer, a PDA computer, a mobile telephone, a micro controller and similar devices. Typically, a computer as defined herein would have a display or communicate with a display. The display could be a touch type of display such that the caregiver could use the display to enter commands or a monitor screen that displays information and images.

The term "three-dimensional (3D) Acquisition system" as used in the present disclosure means a device that acquires data to reconstruct a surface contour. It may include one or more cameras and may include a projector to project a light structure.

### SUMMARY

A system for skin treatment including a treatment energy source that generates a treatment beam. A treatment beam deflecting mechanism is configured to direct the treatment beam to a treated skin area. One or more video cameras are configured to capture a treated skin area and communicate the captured treated skin area image to a processor. The processor is configured to construct, based on a captured treated skin area a three-dimensional (3D) representation of the captured treated skin area. The processor is further configured to control a treatment beam deflecting mechanism to deflect the treatment beam to follow the three-dimensional representation of the captured skin area.

The system for skin treatment includes an infrared imager configured to capture an infrared image of the treated skin area captured by the at least one video camera and communicate the infrared image of the treated skin area to the processor. Based on the infrared image of the treated skin area, the processor is also configured to assess the temperature of adipose tissue located below the treated skin area.

### LIST OF FIGURES AND THEIR BRIEF DESCRIPTION

FIG. 1 is an example of an existing skin treatment laser scanning system;
FIG. 2 is a schematic illustration of a skin treatment energy delivering scanning system according to an example;
FIG. 3 is a schematic illustration of an image displayed on the display of the present system for skin treatment according to an example;
FIG. 4 is an example illustrating dependence of incident electromagnetic radiation beam fluence as a function of the incident angle on a turbid media;
FIG. 5 is an example illustrating dependence of the electromagnetic energy beam fluence distribution as function of the incident angle in the XZ plane;
FIG. 6 is an example illustrating dependence of the electromagnetic radiation beam, such as a laser beam, penetration depth into a turbid media as function of incident angle;
FIG. 7 is a schematic illustration of an example of skin temperature distribution when it is irradiated by a treatment energy radiation;
Fig. 8 illustrates an example of a cryogenic cooling device for cooling large skin area; and
FIG. 9 is an example of a workflow of the skin treatment by the present system for skin treatment.

### DESCRIPTION

Currently, most of the skin treatments by electromagnetic energy and in particular by light are performed by an applicator that when applied to the skin affects an area of 3x3mm² and up to 30x30mm². In order to treat other skin segments or areas, the applicator is repositioned or re-aligned across a large segment of the skin and activated to deliver or couple tissue or skin affecting or treatment energy to this segment of skin. Proper skin treatment and in particular adipose tissue treatment for circumference reduction would provide better results if efficient, homogenous affecting energy delivery over a relatively large skin areas or segments could be performed.

It has been found that it would be advantageous to affect simultaneously or almost simultaneously a large skin area without involving hand motion and applicator repositioning by the caregiver.

The present disclosure suggests an efficient, homogenous and almost simultaneous skin treatment energy delivery apparatus over a relatively large skin areas or segments of skin. Treatment energy beam scanning provided by a deflecting mirror or a rotating polygon supports almost simultaneous delivery of the skin treatment or skin affecting energy across a large area of skin. Overlap of the scanning spot formed by the treatment energy beam along the scanning path removes non-uniformities caused by any none-uniform energy distribution or hot-spots in the treatment energy beam pass. Application of treatment energy by scanning the treatment energy beam makes the skin treatment less dependent or almost not dependent on the caregiver's expertise and reduces the treatment time considerably.

An additional advantage of the treatment energy beam scanning is that it supports variability in position of the scanning spot in all three dimensions/axes. Treatment energy beam spot could be easily positioned at almost any location on the skin in X-Y plane and also moved over relatively large distance in direction of Z axis or depth.

The need to accurately identify the temperature readings or representation of target tissue temperature across the treated skin area under such conditions may represent a serious challenge to any caregiver. The present document also discloses a method of target tissue temperature determination in course of the skin treatment.

As light energy is absorbed rapidly when penetrating the skin, heating of the superficial layers of the skin is inevitable. In order to eliminate the risk of undesired harmful effect of the epidermis and dermis one may use a number of cooling methods such as contact cooling, dynamic-cooling, air-cooling, cryogenic cooling and other known in the art cooling methods. These epidermal protection methods cool the skin in any combination of before, during and after the delivery of light energy to the skin. So the temperature rise within the epidermal and dermal layer is below the threshold of harming the tissue, while still reaching desired treatment temperature in the targeted tissue.

Monitoring the temporal change of the skin temperature could be done by using a thermal camera, IR temperature sensor, ultrasound propagation speed temperature monitoring, contact temperature sensors, non-contact temperature sensing device, or any other means that can be used to assess the temperature in the target tissue. This could be achieved by measuring the amount of heat that has dissipated from the target to the skin and then cooled by either normal air convection of the skin or by taking into account the temporal dynamics of bio-heat equation for the entire treated skin area.

Another advantage of using a scanning treatment energy beam is the ability for continuous control of a large number of variables available in course of the skin treatment. This could include distribution of energy in each of wavelengths of the treatment radiation beam, the spot/area formed by irradiating the skin treatment beam, overlap between two neighbor treatment spots, treatment energy level, exposure duration per unit area or continuous irradiation, selected treatment duration and adaptation to treated skin/tissue area characteristics.

In some examples, the energy dose delivered by a scanning treatment beam spot could be set to cause immediate detectable temperature rise of the treated tissue. In other examples of the apparatus disclosed, the energy dose delivered by a scanning treatment beam spot could be set to cause a slow, immediately not detectable temperature rise of the treated tissue, such that the treated and surrounding tissue is heated but not damaged.

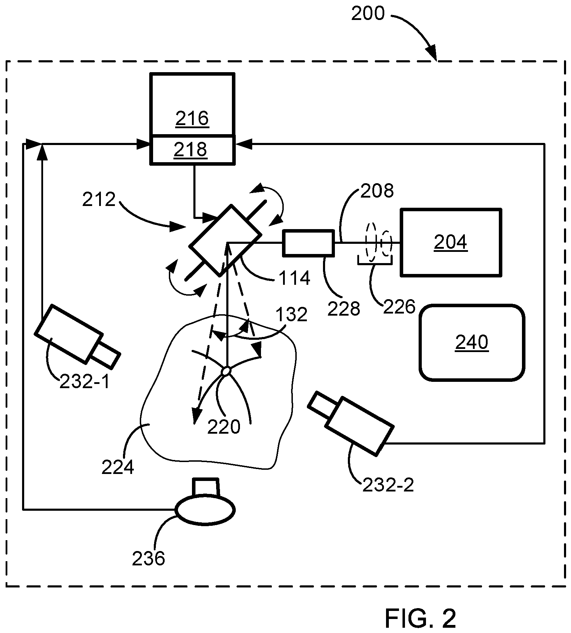

The scanning system could deliver the treatment energy in a continuous or pulsed mode. Uniform scanning treatment beam intensity or fluence distribution and location on the treated skin area among others could be regulated by processor 218 (FIG. 2) that maintains treatment beam scanning speed to facilitate an overlap of at least 30% between two neighbor treatment scanning spots.

In a further example, the treatment beam scanning speed could be set to match the thermal relaxation time and perfusion rate of the targeted skin/tissue, such as dependent on the size of the treated area and desired temperature to be maintained, or a homogenous desired skin temperature is maintained for a certain volume of targeted tissue.

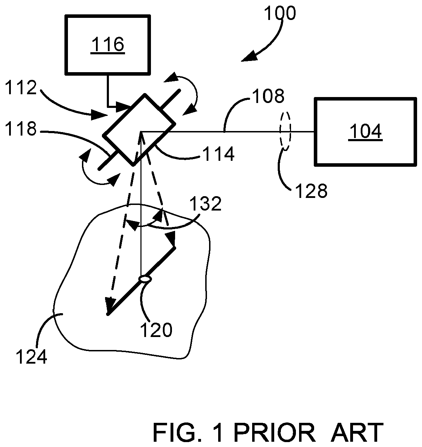

Reference is made to FIG. 1 which is an example of an existing skin treatment laser scanning system. Skin treatment system 100 includes a treatment energy source 104 that provides a treatment radiation beam 108. Treatment radiation beam 108 impinges on a treatment beam deflecting or scanning mechanism 112 that could be one or more of flat or concave deflecting mirrors, a scanning polygon, a holographic disk and a combination of the above. A control module 116 controls position of the scanning mechanism 112 and is configured to locate treatment scanning spot 120 at any coordinate in X-Y scanning plane 124. System 100 could also include a lens 128 that could be a flat field lens or a regular lens or lens array depending on the length or angle of the treatment beam scanning and the depth profile of the treated skin. In some examples lens 128 could be a beam expander\reducer matching the spot size on the skin with a treatment plan or protocol.

When treatment energy beam 108 is directed to scan across the skin the actual spot size produced by the treatment beam intensity may change due to change in the incidence angle and the skin curvature at any location on skin, the fluence of the treatment energy changed in order to compensate and reach the desired treatment energy intensity by temporarily increasing the source power.

FIG. 2 is a schematic illustration of a skin treatment system according to an example. Skin treatment system 200 includes a treatment energy source 204 that is configured to generate a treatment radiation beam 208. Treatment radiation or treatment energy source 204 could be a semiconductor optical energy source such as laser diode, VCSEL, an assembly of laser diodes or bars, a solid state laser, a fiber laser, or other laser energy source with suitable power and wavelengths. Treatment energy source 204 could operate at wavelength of visible to NIR (450-2000nm) and provide treatment energy radiation power of 1W to 17kW. The treatment energy source 204 in FIG. 2 can be a broad spectrum light source of either coherent or non-coherent radiation such as a Xe, Kr, W, Quartz-Iodine lamps or a LED. Treatment radiation beam or simply treatment beam 208 impinges on a treatment beam deflecting mechanism 212 that could be one or more deflecting mirrors, a scanning polygon, a holographic scanning system and a combination of the above. In one example, the treatment beam deflecting mechanism 212 could be a two dimensional beam deflecting or scanning mechanism, In a further example, the treatment energy beam deflecting mechanism 212 could deflect the treatment beam along one axis X or Y and a linear movement or displacement of the treatment beam deflecting mechanism could be used to scan in the other direction.

A computer 216 that includes a processor 218 which controls position of the scanning mechanism 212 and is configured to locate treatment beam scanning spot 220 at any coordinate in scanning plane 224. Processor 218 also controls the scanning mechanism 212 to produce a plurality of treated skin area scanning patterns and further controls the scanning speed of treatment scanning spot 220 and treatment energy source operation time. Control module 216 and in particular processor 218 controls all elements of system 200 including operation time and parameters of treatment energy source 204. It has been noted above that the human or animal skin usually has a three-dimensional contour or profile. In one example, system 200 includes a dynamic focus module 228, such as HPLK or Pro-series module, commercially available from Cambridge Technology, Inc., Bedford, MA 01730 U.S.A. However, in the current disclosure the Dynamic Focus Module (DFM) is used to follow the three-dimensional contour or profile of the human skin and not to flatten the X-Y plane. In order to compensate for any change in the curvature of the skin and deliver the prescribed fluence or power dose, the treatment radiation beam divergence could be changed. The change in divergence would cause a change in the spot size and changes to the treatment radiation intensity delivered by the scanning spot could be introduced. By changing treatment radiation beam 208 divergence, the diameter of the scanning spot 220 could be changed up to ten times or even more. The diameter of spot 220 could change for example, from 5 to 30mm.

The scanning or treatment energy beam sweep angle and 3D (three dimensional) nature of human body distort to certain degree the scanning treatment spot shape and cause a treatment beam intensity roll-off at peripheral treatment beams. Scanning treatment energy spot shape distortion could be compensated among others by changing the size of the scanning spot and/or the amount of fluence delivered into the treatment energy radiation beam. The amount of fluence or intensity delivered into the treatment radiation beam could be compensated by providing a treatment intensity roll-off look-up table or by calculating the change in energy in real-time. Based on the treatment intensity roll-off look-up table processor 218 adjusts the deflected treatment energy beam intensity to maintain a roll-off the treatment energy beam intensity or fluence of less than 10% (10 percent). The look-up table is calculated based on the skin 3D contour and the treatment energy beam incidence angle that is usually less than 30 degrees. The scanning system could deliver the treatment energy in a continuous or pulsed mode. Uniform scanning treatment radiation beam distribution on the treated skin area among others could be regulated by processor 218 (FIG. 2) that maintains treatment beam scanning speed to facilitate an overlap of at least 30% between two neighbor treatment scanning spots.

System 200 further includes a 3D acquisition system 232 (For example, video cameras 232-1 and 232-2) configured to capture a treated skin area or segment and communicate the captured treated skin area image to processor 218. The 3D acquisition system 232 also communicates the captured image or images to processor 218, which based on the communicated image or images is configured to reconstruct/determine the three-dimensional (3D) contour of the treated segment or area of the human body. The 3D acquisition system 232 could be equipped with an optical zoom system supporting imaging of different sizes of the treated skin area or segment. Processor 218 is also configured to construct based on the captured skin area a three-dimensional representation or topography of the captured skin area. Processor 218 is further configured to control the treatment energy beam deflecting mechanism to deflect the treatment energy beam to follow the topography or three-dimensional representation of the captured skin area.

System 200 further includes one or more infrared (IR) cameras or imagers 236 configured to provide processor 218 with a thermal image of the skin affected by the treatment energy radiation. Infrared imager 236 could be almost any infrared camera supporting temperature sensitivity of 1°K or better. Infrared imager supports non-contact and non-invasive skin temperature measurement. IR imager or camera 236 could have a resolution sufficient to support imaging of an area of the treated skin segment with dimensions of 30x30cm² or smaller. Processor 218 is configured to receive the thermal image indicating temperature distribution on the surface of the currently treated by the treatment energy beam skin segment and determine the temperature of the currently treated skin area or segment. Physical properties of human tissue are known and relatively well established. Temperature distribution below the skin surface can be calculated based on skin surface temperature and finite elements analyses, using the Bio-heat equation or other suitable numerical and statistical methods known for solving the different heat distribution equations.
(For Bio-heat equation details see H.H. Pennes, Analysis of Tissue and Arterial Blood Temperature in the Resting Human Forearm, J. Appl. Phys. vol. 1, pp. 93-122, 1948).

Without forming part of the invention, methods of assessing temperature inside the body by analyzing the radiation reflection spectrum or any other known in the art method could be used to assess temperature of the adipose tissue located below the skin. Real-time temperature monitoring facilitates safe and effective skin treatment.

Imager or infrared camera 236 could be equipped by an optical zoom system supporting imaging of a large area or segment of the treated skin or a small area of the treated skin segment.

System 200 further includes a display 240. Processor 218 based on the signals received from the infrared imager 236 continuously or at predetermined intervals updates the displayed thermal image of the treated skin segment. Based on the signals received from the 3D acquisition system 232, processor 218 issues corrections to the treatment energy spot and treatment energy beam location to follow the skin contour.

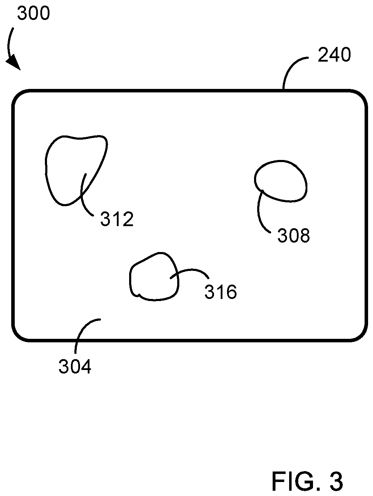

Display 240 is configured to receive from processor 218 the thermal image of the treated skin segment and to display the temperature of the skin segment or thermal map 300 (FIG. 3) of the treated by the skin treatment energy skin segment. Display of the image captured by the 3D acquisition system facilitates visual control of the process by the caregiver. The image provided by 3D acquisition system 232 could include area larger than the treated skin areas and include surrounding the skin treatment area skin segments.

FIG. 3 is a schematic illustration of an image displayed on the display of the present system for skin treatment according to an example. Image 300 shows area 304 with a homogenous skin temperature that could be a desired treatment temperature. Areas 308 - 316 illustrate segments of skin having temperature different from the desired treatment temperature. The temperature could be higher or lower than the desired treatment temperature and each area 308 - 316 could have a different temperature.

Display 240 is also configured to receive from computer 218 processor 216 (FIG. 2) the thermal image of the treated skin segment and the current or most recent location of the scanning treatment energy spot 220, within the treated by the skin treatment energy skin segment.

In some examples control of the treatment process and of the scanning system could be simplified by forming a specific scanning geometry, for example, limiting the treatment energy beam incidence angle to 20, 15 or 10 degrees. At such treatment energy beam incident angles, scanned treatment energy power is almost constant and skin topography does not change significantly.

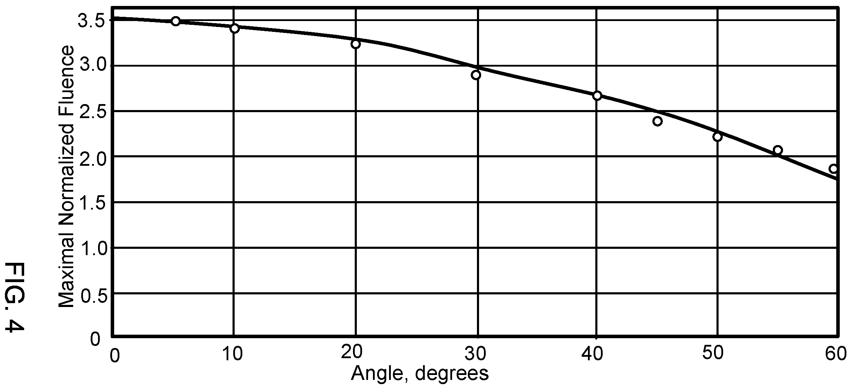

FIG. 4 is an example illustrating dependence of incident electromagnetic radiation beam fluence as a function of the incident angle on a turbid media. The electromagnetic radiation could be a laser beam. Turbid media was simulating human tissue or skin and was produced/simulated by different concentration of milk in water. The figure (FIG. 4) shows that in highly concentrated turbid media at incidence angles from 0 degrees to about 30 degrees the fluence of the laser beam does not significantly change.

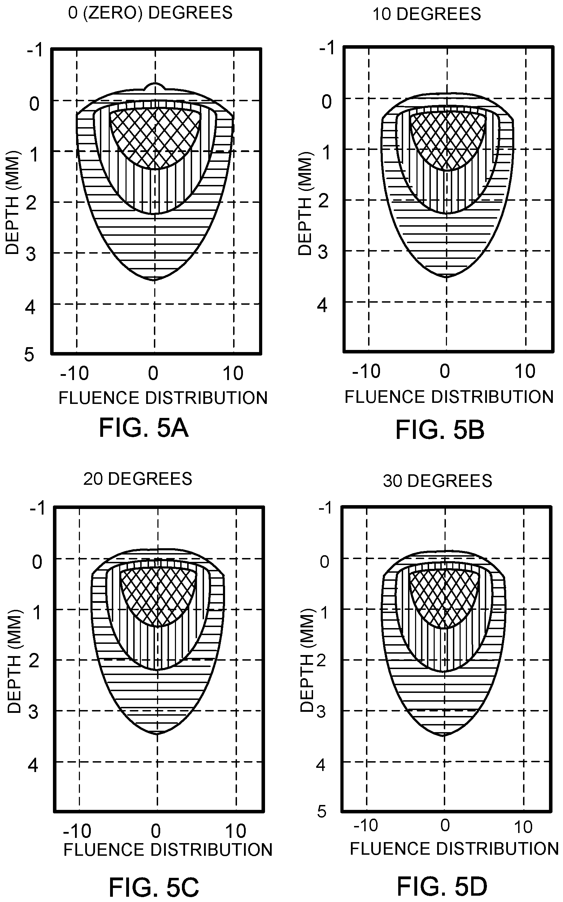

FIG. 5 is an illustration of the incident electromagnetic energy beam fluence distribution in the XZ plane (in the depth of the turbid media) at different electromagnetic energy beam incident angles. The electromagnetic energy beam had a radius R=8mm. The crosshatched area is an area with maximal electromagnetic energy beam fluence. The fluence distribution pattern does not manifests visible asymmetry.

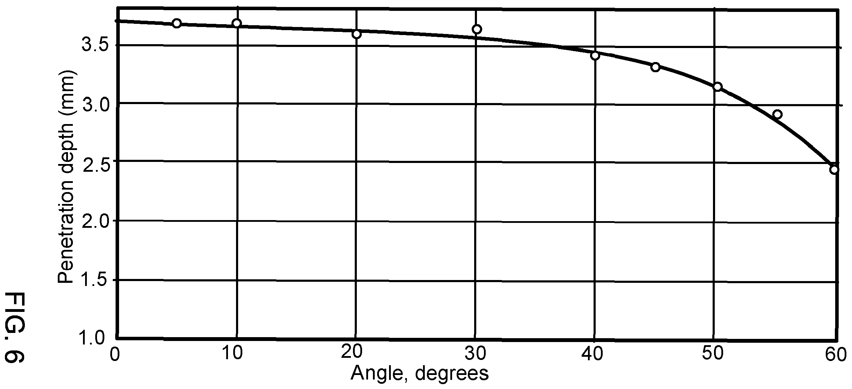

FIG. 6 is an example illustrating dependence of the electromagnetic radiation beam, such as a laser beam, penetration depth into a turbid media as a function of incidence angle. The figure clear shows that for incidence angles ranging from 0° (zero degrees) to about 40° (degrees) the penetration depth of the laser beam into a turbid media is weakly dependent on the laser beam incidence angle.

In FIG. 4 and FIG. 6 solid line marks measured values and round dots mark calculated values. Experimental results have been obtained using a Nd:YAG (532 nm) and a laser diode (800 nm) electromagnetic radiation.

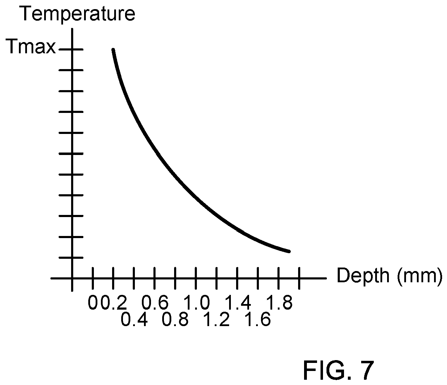

FIG. 7 is a schematic illustration of an example of skin temperature distribution when it is irradiated by a treatment radiation. Treatment radiation scanning beam scans the skin surface from which the treatment energy penetrates into deeper skin layers and heats for example, hair roots, adipose tissue and collagen. Prolonged heating, for example 30 to 50min of the target skin areas (e. g. hair roots, adipose tissue) and maintenance of the target areas at a constant relatively low skin temperature of for example, 40 degrees Celsius, causes the desired skin treatment effect. The effect could be hair removal, adipose tissue reduction, wrinkle reduction and other. As it has been illustrated in FIG. 3 some skin areas could have a temperature higher or lower than the desired treatment temperature. Skin areas with higher temperature could be cooled.

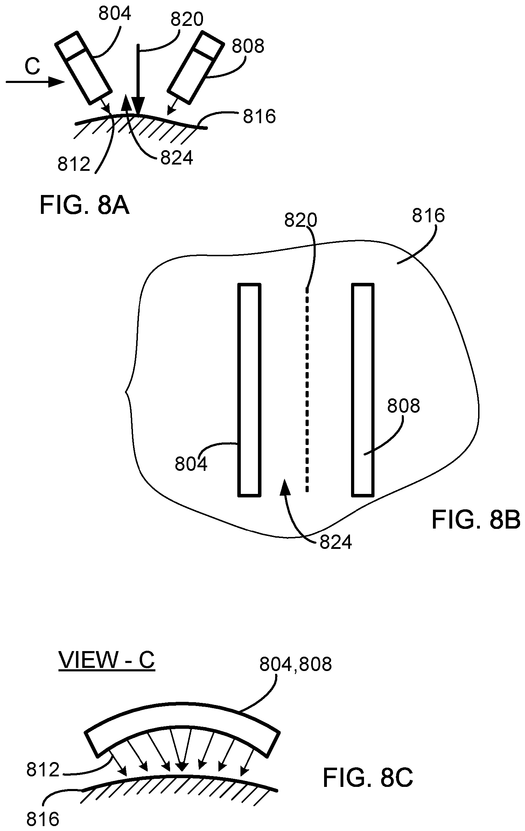

Fig. 8 illustrates an example of a cryogenic cooling device for cooling large skin area. Cryogenic cooling device includes two bars 804 and 808 (FIG. 8A - 8B) spaced apart with a plurality of nozzles through which cryogenic gas or cold air, schematically shown in FIG. 8C by arrows 812, is directed to skin surface 816. Treatment energy radiation 820 is directed into space 824 between bars 804 and 808. Bars 804 and 808 could move in a synchronous or asynchronous movement mode following the treatment radiation beam and changing width of space 824 between bars 804 and 808. Bars 804 and 808 could be of straight shape or a have a type of arched or curvilinear shape (FIG. 8C) to approximate the shape of the treated skin area.

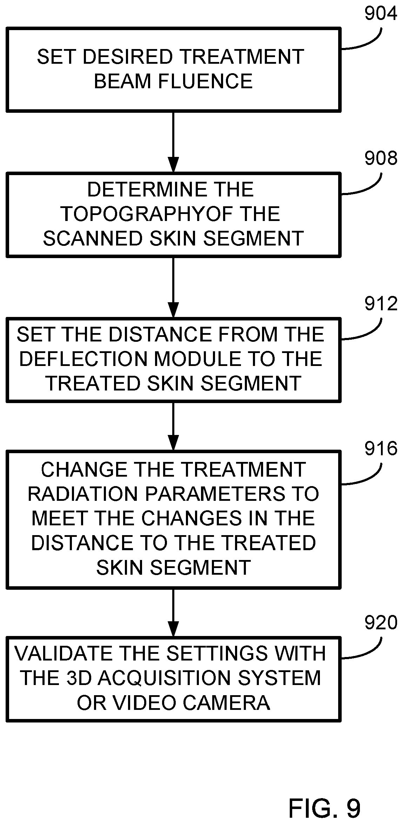

FIG. 9 is an example of a workflow of the skin treatment by the present system for skin treatment. Initially, operator or caregiver sets the desired treatment beam fluence (Block 904). Proper fluence values could be achieved by setting treatment radiation power from 1.0W to 10kW. Concurrently treatment radiation pulse duration is set to be 1msec to 1 sec or CW operation. At block 908 the operator determines topography or 3D profile of the scanned surface. Given the topography of the scanned surface, it is possible to set the distance from the deflection module to the treated skin segment (Block 912). The operator sets the treatment radiation power and other treatment radiation parameters to meet the change in the distance to the treated skin segment (Block 916). In some examples in addition to change in the distance to the treated skin segment treatment changes to beam divergence or focal length of lens 128 also could take place. Changes in beam divergence or focal length could control the size of the scanning spot 220 (FIG. 2) formed by the treatment beam. Spot 220 could change from 5 to 30mm. Upon completion of the settings a video camera 232 could be used to validate (Block 920) the distance to the treated skin surface.

According to another example, control of the distance between the scanning mechanism and the treated skin surface could be performed based on the dimensions/size of the treated body. Image sensors, such as video cameras 232 (FIG. 2) could be adapted to provide the desired information to processor 218 that would execute a proper algorithm supporting determination of the dimensions/size of the treated body.

In some examples the treatment process settings and control could be simplified by using prepared ahead of time standard skin treatment procedures parameters. The procedures could be stored in the memory as a Look-up-Table (LUT) of computer 216 (FIG. 2), which controls the process of the skin treatetment.

While the apparatus has been particularly shown and described with references to some examples thereof, it will be understood by those skilled in the art that various changes in form and details may be made therein without departing from the scope of the apparatus encompassed by the appended claims.

## Claims

1. A system comprising:
at least one treatment energy source (104, 204) for generating a treatment beam (108, 208);
at least one treatment beam (108, 208) deflecting mechanism (112, 212) configured to direct the treatment beam (108, 208) to a treated skin area;
at least one video camera (236) configured to capture a treated skin area and communicate captured treated skin area image to a processor (218); and
a processor (218) configured to construct, based on a captured treated skin area a three-dimensional representation of the captured treated skin area and wherein the processor (218) is further configured to control a treatment beam deflecting mechanism (112, 212) to deflect the treatment beam (108, 208) to follow the three-dimensional representation of the captured skin area,
**characterized in that** the system further comprises:
a treatment beam (108, 208) intensity roll-off look-up table, wherein the processor (218) is configured to adjust a deflected treatment beam (108, 208) intensity based on the treatment beam intensity roll-off look-up table.

2. The system according to claim 1 further comprising an infrared imager (236) configured to capture an infrared image (300) of the treated skin area captured by the at least one video camera (236) and communicate the infrared image (300) of the treated skin area to the processor (218) and wherein the processor (218) is also configured based on the infrared image (300) of the treated skin area to assess at least temperature of adipose tissue located below the treated skin area.

3. The system according to claim 1 wherein scanning angle of the treatment beam (108, 208) is less than 30 degrees and wherein treatment beam (108, 208) intensity roll-off is less than 10 percent.

4. The system according to claim 1 wherein the processor (218) controls a treatment beam deflecting mechanism (112, 212) to produce a plurality of treated skin area scanning patterns and wherein the processor (218) also controls a treatment beam (108, 208) scanning speed.

5. The system according to claim 1 wherein treatment beam (108, 208) intensity and scanning speed are selected to support temperature of adipose tissue located below the treated skin area at at least 40 degrees Celsius.

6. The system according to claim 1 wherein a treatment beam (108, 208) intensity varies along a scanning angle.

7. The system according to claim 1 wherein the treatment energy source delivers treatment energy in a continuous or pulsed mode, and preferably wherein a temperature sensing device is a non-contact temperature measuring device.

8. The system according to claim 1 wherein the treatment beam deflecting mechanism (112, 212) is at least one of a group of elements consisting of a flat mirror, concave mirror, holographic element and a rotating polygon.

9. The system according to claim 1 wherein the scanning treatment beam forms a scanning spot (120, 220) on the treated skin area and wherein the processor (218) maintains treatment beam (108, 208) scanning speed to maintain an overlap of at least 30% between two neighbor treatment spots.

10. The system according to claim 1 wherein a treatment beam (108, 208) scanning speed is set:
to match a thermal relaxation time and perfusion rate of a targeted skin and/or according to size of the treated area and desired temperature to be maintained.

## Patentansprüche

1. System, das aufweist:
wenigstens eine Behandlungsenergiequelle (104, 204) zum Erzeugen eines Behandlungsstrahls (108, 208);
wenigstens einen Behandlungsstrahl (108, 208) - Umlenkungsmechanismus (112, 212), der ausgestaltet ist, den Behandlungsstrahl (108, 208) auf einen behandelten Hautbereich zu richten;
wenigstens eine Videokamera (236), die ausgestaltet ist, den behandelten Hautbereich zu erfassen und erfasste Bilder eines behandelten Hautbereichs zu einem Prozessor (218) zu übermitteln; und
einen Prozessor (218), der ausgestaltet ist, basierend auf einem erfassten behandelten Hautbereich eine dreidimensionale Darstellung des erfassten behandelten Hautbereichs zu konstruieren, wobei der Prozessor (218) weiter ausgestaltet ist, einen Behandlungsstrahl - Umlenkungsmechanismus (112, 212) zu steuern, um den Behandlungsstrahl (108, 208) umzulenken, um der dreidimensionalen Darstellung des erfassten Hautbereichs zu folgen,
**dadurch gekennzeichnet, dass** das System weiter aufweist:
eine Behandlungsstrahl (108, 208) - Intensitätsablaufwertetabelle, wobei der Prozessor (218) ausgestaltet ist, eine Intensität eines umgelenkten Behandlungsstrahls (108, 208) basierend auf der Behandlungsstrahl-Intensitätsablaufwertetabelle einzustellen.

2. System nach Anspruch 1, das weiter einen Infrarotbildgeber (236) aufweist, der ausgestaltet ist, ein Infrarotbild (300) des behandelten Hautbereichs, der durch die wenigstens eine Videokamera (236) erfasst wird, zu erfassen und das Infrarotbild (300) des behandelten Hautbereichs zu dem Prozessor (218) zu übermitteln, wobei der Prozessor (218) auch ausgestaltet ist, basierend auf dem Infrarotbild (300) des behandelten Hautbereichs wenigstens eine Temperatur von adipösem Gewebe, das unterhalb des behandelten Hautbereichs positioniert ist, abzuschätzen.

3. System nach Anspruch 1, wobei ein Abtastwinkel des Behandlungsstrahls (108, 208) kleiner als 30 Grad ist und wobei der Behandlungsstrahl (108, 208) - Intensitätsablauf kleiner als 10 Prozent ist.

4. System nach Anspruch 1, wobei der Prozessor (218) einen Behandlungsstrahl - Umlenkungsmechanismus (112, 212) steuert, um eine Vielzahl von Abtastmustern eines behandelten Hautbereichs zu erstellen, und wobei der Prozessor (218) auch eine Behandlungsstrahl (108, 208) - Abtastgeschwindigkeit steuert.

5. System nach Anspruch 1, wobei eine Behandlungsstrahl (108, 208) - Intensität und eine Abtastgeschwindigkeit so gewählt werden, dass eine Temperatur von adipösem Gewebe, das unterhalb des behandelten Hautbereichs positioniert ist, auf wenigstens 40 Grad Celsius gestützt wird.

6. System nach Anspruch 1, wobei sich eine Behandlungsstrahl (108, 208) - Intensität entlang eines Abtastwinkels ändert.

7. System nach Anspruch 1, wobei die Behandlungsenergiequelle Behandlungsenergie in einem durchgehenden oder gepulsten Modus abgibt und wobei vorzugsweise ein Temperatursensorgerät ein Nicht-Kontakt-Temperaturmessgerät ist.

8. System nach Anspruch 1, wobei der Behandlungsstrahl-Umlenkungsmechanismus (112, 212) wenigstens einer aus einer Gruppe von Elementen ist, die aus einem flachen Spiegel, einem konkaven Spiegel, einem holographischen Element und einem rotierenden Polygon besteht.

9. System nach Anspruch 1, wobei der abtastende Behandlungsstrahl einen Abtastungspunkt (120, 220) auf dem behandelten Hautbereich bildet und wobei der Prozessor (218) eine Behandlungsstrahl (108, 208) - Abtastgeschwindigkeit beibehält, um einen Überlapp von wenigstens 30 % zwischen zwei benachbarten Behandlungspunkten beizubehalten.

10. System nach Anspruch 1, wobei eine Behandlungsstrahl (108, 208) - Abtastgeschwindigkeit gesetzt wird:
um eine thermische Relaxationszeit und Perfusionsrate einer angezielten Haut und/oder gemäß einer Größe des behandelten Bereichs anzupassen und eine gewünschte Temperatur aufrechtzuerhalten.

## Revendications

1. Système comprenant :
au moins une source d'énergie thérapeutique (104, 204) permettant de produire un faisceau thérapeutique (108, 208) ;
au moins un mécanisme de déviation (112, 212) de faisceau thérapeutique (108, 208) conçu pour diriger le faisceau thérapeutique (108, 208) vers une zone de peau traitée,
au moins une caméra vidéo (236) conçue pour capturer une zone de peau traitée et communiquer une image de zone de peau traitée capturée à un processeur (218) ; et
un processeur (218) conçu pour construire, sur la base d'une zone de peau traitée capturée, une représentation tridimensionnelle de la zone de peau traitée capturée et dans lequel le processeur (218) est en outre conçu pour commander un mécanisme de déviation de faisceau thérapeutique (112, 212) pour dévier le faisceau thérapeutique (108, 208) afin de suivre la représentation tridimensionnelle de la zone de peau capturée,
caractérisé ce que le système comprend en outre :
une table de consultation d'affaiblissement d'intensité de faisceau thérapeutique (108, 208), dans lequel le processeur (218) est configuré pour ajuster une intensité de faisceau thérapeutique dévié (108, 208) sur la base de la table de consultation d'affaiblissement d'intensité de faisceau de traitement.

2. Système selon la revendication 1, comprenant en outre un imageur infrarouge (236) configuré pour capturer une image infrarouge (300) de la zone de peau traitée capturée par la au moins une caméra vidéo (236) et communiquer l'image infrarouge (300) de la zone de peau traitée au processeur (218) et dans lequel le processeur (218) est également configuré sur la base de l'image infrarouge (300) de la zone de peau traitée pour évaluer au moins la température de tissu adipeux situé en dessous de la zone de peau traitée.

3. Système selon la revendication 1, dans lequel l'angle de balayage du faisceau thérapeutique (108, 208) est inférieur à 30 degrés et dans lequel l'affaiblissement d'intensité du faisceau thérapeutique (108, 208) est inférieur à 10%.

4. Système selon la revendication 1, dans lequel le processeur (218) commande un mécanisme de déviation de faisceau thérapeutique (112, 212) pour produire une pluralité de motifs de balayage de zone de peau traitée et dans lequel le processeur (218) commande également une vitesse de balayage de faisceau thérapeutique (108, 208).

5. Système selon la revendication 1, dans lequel l'intensité et la vitesse de balayage du faisceau thérapeutique (108, 208) sont sélectionnées pour supporter la température du tissu adipeux situé au-dessous de la zone de peau traitée à au moins 40 degrés Celsius.

6. Système selon la revendication 1, dans lequel une intensité de faisceau thérapeutique (108, 208) varie le long d'un angle de balayage.

7. Système selon la revendication 1, dans lequel la source d'énergie thérapeutique délivre de l'énergie thérapeutique dans un mode continu ou pulsé, et de préférence dans lequel un dispositif de détection de température est un dispositif de mesure de température sans contact.

8. Système selon la revendication 1, dans lequel le mécanisme de déviation de faisceau thérapeutique (112, 212) est au moins l'un d'un groupe d'éléments comprenant un miroir plat, un miroir concave, un élément holographique et un polygone rotatif.

9. Système selon la revendication 1, dans lequel le faisceau thérapeutique de balayage forme un point de balayage (120, 220) sur la zone de peau traitée et dans lequel le processeur (218) maintient la vitesse de balayage du faisceau thérapeutique (108, 208) pour maintenir un chevauchement d'au moins 30 % entre deux points thérapeutique voisins.

10. Système selon la revendication 1, dans lequel une vitesse de balayage de faisceau thérapeutique (108, 208) est établie :
pour correspondre à un temps de relâchement thermique et à une vitesse de perfusion d'une peau ciblée et/ou en fonction de la taille de la zone traitée et de la température souhaitée à maintenir.
